# FASCICULE DE BREVET EUROPEEN

(11) **EP 3 651 840 B1**
(45) Date de publication et mention de la délivrance du brevet: **12.05.2021**
(21) Numéro de dépôt: 18752579.5
(22) Date de dépôt: 10.07.2018
(51) Int. Cl.: A61M 15/00

(54) **DISPOSITIF DE DISTRIBUTION DE PRODUIT FLUIDE**
VORRICHTUNG ZUR VERTEILUNG EINES FLÜSSIGPRODUKTS
FLUID PRODUCT DISTRIBUTION DEVICE

(30) Priorité: 11.07.2017 FR 1756579
(43) Date de publication de la demande: 20.05.2020
(73) Titulaire: Aptar France SAS, 27110 Le Neubourg (FR)
(72) Inventeur: HELMLINGER, Michael, 78315 Radolfzell (DE); KÖRNER, Joachim, 88690 Uhldingen-Mühlhofen (FR)
(74) Mandataire: CAPRI
(86) Numéro de dépôt international: PCT/FR2018/051733
(87) Numéro de publication internationale: WO 2019/012221

(56) Documents cités:
- WO-A1-01/41846
- WO-A1-2009/077697
- WO-A1-2013/175120
- WO-A1-2013/178951
- WO-A2-2008/012458
- FR-A1- 2 936 424
- US-A1- 2005 174 216
- US-A1- 2005 177 275

## Description

La présente invention concerne un dispositif de distribution de produit fluide, et plus particulièrement un inhalateur de poudre sèche.

Les inhalateurs sont bien connus dans l'état de la technique. Il en existe différentes sortes.

Un premier type d'inhalateur contient un réservoir recevant une multitude de doses de poudre, l'inhalateur étant pourvu de moyens de dosage permettant à chaque actionnement de séparer une dose de cette poudre du réservoir pour l'amener dans un conduit d'expulsion afin d'être distribué à l'utilisateur.

Des inhalateurs comportant des réservoirs individuels, tels que des capsules, qui sont à charger dans l'inhalateur juste avant l'utilisation de celui-ci ont également été décrits dans l'état de la technique. L'avantage de ces dispositifs est qu'il n'est pas nécessaire de stocker l'ensemble des doses à l'intérieur de l'appareil, de sorte que celui-ci peut être de dimension réduite. Par contre, l'utilisation est plus complexe, puisque l'utilisateur est obligé de charger une capsule dans l'inhalateur avant chaque utilisation.

Un autre type d'inhalateur consiste à disposer les doses de poudre dans des réservoirs individuels pré-dosés, puis d'ouvrir un de ces réservoirs à chaque actionnement de l'inhalateur. Cette mise en œuvre assure une meilleure étanchéité de la poudre, puisque chaque dose n'est ouverte qu'au moment de son expulsion. Pour réaliser ces réservoirs individuels, diverses variantes ont déjà été proposées, telle qu'une bande de blisters allongée ou des blisters disposés sur un disque circulaire rotatif.

Tous les types d'inhalateurs décrits ci-dessus et existants présentent des avantages et des inconvénients liés à leur structure et à leur fonctionnement.

Ainsi, avec certains inhalateurs, se pose le problème de la précision et de la reproductibilité du dosage à chaque actionnement.

De même, l'efficacité de la distribution, c'est-à-dire la partie de la dose qui pénètre effectivement dans les poumons de l'utilisateur pour avoir un effet thérapeutique bénéfique, est également un problème qui se présente avec un certain nombre d'inhalateurs.

Une solution pour résoudre ce problème spécifique a été de synchroniser l'expulsion de la dose avec l'inhalation du patient. A nouveau, ceci pouvait générer des inconvénients, à savoir que généralement dans ce type de dispositif, la dose est d'abord chargée dans un conduit d'expulsion avant l'inhalation, puis l'expulsion est synchronisée avec l'inhalation. Ceci signifie que si l'utilisateur laisse tomber, secoue ou manipule de manière non souhaitée ou inadaptée l'inhalateur entre le moment où il a chargé la dose (soit à partir d'un réservoir multidoses, soit à partir d'un réservoir individuel) et au moment où il inhale, il risque de perdre toute ou partie de cette dose, celle-ci pouvant se répartir à l'intérieur de l'appareil. Dans ce cas il peut se présenter un gros risque de surdosage lors de la prochaine utilisation du dispositif. L'utilisateur qui se rendra compte que sa dose n'est pas complète chargera une nouvelle dose dans l'appareil, et lors de l'inhalation de cette nouvelle dose, une partie de la dose précédente perdue dans l'appareil pourrait être alors expulsée en même temps que la nouvelle dose, provoquant un surdosage. Selon les traitements envisagés, ce surdosage peut être très néfaste et c'est une exigence de plus en plus forte des autorités de tous les pays de limiter au maximum ce risque de surdosage.

Un autre problème qui peut se poser concerne l'assemblage de certaines pièces, notamment mobiles, qui doivent supporter des contraintes importantes en fonctionnement, et pour lesquels l'assemblage doit être particulièrement fiable pour éviter tout risque de dysfonctionnement. Avec la petite taille de certaines pièces et des possibles déformations issues de moulage et/ou du conditionnement de composants plastiques, il peut être compliqué de garantir une telle fiabilité d'assemblage. Des assemblages étanches, notamment dans des inhalateurs utilisant un système de déclenchement par l'inhalation, sont souhaitables, notamment pour éviter toute perte de charge lors de l'inhalation, mais aussi pour assurer la répétabilité du seuil de déclenchement. Ceci améliore le confort d'utilisation des personnes malades et permet de ne pas perturber les habitudes des patients causées par d'éventuelles variations d'un inhalateur à l'autre.

Par ailleurs, il est connu d'utiliser un compteur ou indicateur de doses pour informer l'utilisateur du nombre de doses distribuées ou restant à distribuer. Ces compteurs ou indicateurs sont généralement mécaniques. Un inconvénient classique avec ces compteurs mécaniques est qu'ils sont encombrants, augmentant d'autant la dimension de l'inhalateur lui-même. De plus, l'affichage est très petit et souvent difficile à lire, notamment pour les personnes âgées. Ceci est notamment vrai pour les compteurs destinés à compter un nombre élevé de doses, par exemple 30 ou 60 doses. Un autre problème lié au comptage de doses concerne les compteurs ou indicateurs dont l'actionnement n'est pas directement lié à la distribution d'une dose de poudre, mais à une phase d'actionnement telle que le chargement de la dose à distribuer ou similaire. Si pour une quelconque raison l'utilisateur n'inhale finalement pas la dose chargée, celle-ci aura malgré tout été comptée.

Les documents US6179164, US2005174216, FR2936424, FR2936425, WO02085281, WO2008012458, WO2009077697 et WO2011154659 décrivent des dispositifs de l'art antérieur.

La présente invention a pour but de fournir un dispositif de distribution de produit fluide, en particulier un inhalateur de poudre sèche, qui ne reproduit pas les inconvénients susmentionnés.

En particulier, la présente invention a pour but de fournir un tel dispositif qui soit fiable à l'assemblage et en utilisation, garantissant une précision et une reproductibilité du dosage à chaque actionnement, fournissant un rendement optimal quant à l'efficacité du traitement, en permettant de distribuer une part importante de la dose au niveau des zones à traiter, en particulier les poumons, évitant de manière sûre et efficace les risques des surdosages, de dimensions aussi petites que possibles, tout en garantissant une étanchéité et une intégrité absolue de toutes les doses jusqu'à leur expulsion.

La présente invention a aussi pour but de fournir un tel dispositif qui assure un comptage fiable et précis à chaque actionnement, en empêchant tout risque de sous-comptage et/ou de sur-comptage.

Par ailleurs, la présente invention a pour but de fournir un tel dispositif qui soit simple et peu coûteux à fabriquer et à assembler.

La présente invention a donc pour objet un dispositif de distribution de produit fluide, comportant un corps principal, ledit dispositif comportant:
- au moins un réservoir individuel contenant une dose unique de produit fluide, tel que de la poudre,
- des moyens d'ouverture pour ouvrir un réservoir individuel à chaque actionnement du dispositif,
- un embout buccal définissant un orifice de distribution,
- des moyens de support mobiles adaptés à déplacer un réservoir individuel contre lesdits moyens d'ouverture à chaque actionnement, lesdits moyens de support mobiles étant déplaçables entre une position de non-distribution et une position de distribution, lesdits moyens de support mobiles étant sollicités vers leur position de distribution par des moyens élastiques, tel qu'un ressort ou une lame élastique, et étant retenus en position de non-distribution par des moyens de blocage,
- un système de déclenchement par l'inhalation qui comporte une chambre d'air déformable coopérant avec ledit orifice de distribution, un élément déclencheur coopérant d'une part avec ladite chambre d'air et d'autre part avec lesdits moyens de blocage, de sorte que lors d'une inhalation à travers ledit orifice de distribution, ladite chambre d'air est déformée et ledit élément déclencheur libère lesdits moyens de blocage, de sorte que lors d'une inhalation, un réservoir est déplacé contre et ouvert par lesdits moyens d'ouverture,
ledit dispositif de distribution de produit fluide comportant un dispositif électronique de comptage ou d'indication de doses comprenant un capteur adapté à détecter le déplacement lors ou après l'inhalation de l'utilisateur desdits moyens de support mobiles (50).

Avantageusement, ledit capteur est actionné par lesdits moyens de support mobiles lors de leur déplacement entre ladite position de non-distribution et ladite position de distribution.

En variante, ledit capteur est actionné par lesdits moyens de support mobiles lors de leur déplacement entre ladite position de distribution et ladite position de non-distribution.

Avantageusement, le dispositif comporte une bande allongée de réservoirs individuels coopérant avec des premiers moyens de déplacement adaptés à faire avancer ladite bande après chaque actionnement, lesdits premiers moyens de déplacement comportant une roue de guidage montée rotative sur lesdits moyens de support mobiles.

Avantageusement, ledit capteur est actionné par la rotation de ladite roue de guidage.

Avantageusement, ledit dispositif électronique de comptage ou d'indication de doses comprend un écran, notamment du type LCD, une source d'alimentation, telle qu'une pile ou un accumulateur, et un circuit imprimé.

Avantageusement, ledit capteur, lorsqu'il est actionné, transmet un signal audit circuit imprimé, qui va commander la modification de l'affichage dudit écran.

Avantageusement, le dispositif comporte un organe d'armement déplacé vers une position chargée lors de l'ouverture du dispositif de distribution de produit fluide, ledit dispositif électronique de comptage ou d'indication de doses comprenant un actionneur pourvu d'un connecteur, et ledit organe d'armement comportant une extension adaptée à coopérer, dans la position chargée dudit organe d'armement, avec ledit connecteur pour activer le dispositif électronique de comptage ou d'indication de doses.

Avantageusement, ledit dispositif électronique de comptage ou d'indication de doses comprend une mémoire adaptée à stocker les informations générées par ledit capteur.

Avantageusement, ledit dispositif électronique de comptage ou d'indication de doses comprend des moyens de transmission de données sans fil, notamment du type Bluetooth®, en particulier Bluetooth® à basse énergie (BLE).

Avantageusement, ledit dispositif électronique de comptage ou d'indication de doses comprend d'autres capteurs associés audit capteur, notamment du type accéléromètre, pour détecter l'orientation et/ou les mouvements de l'utilisateur lors de l'actionnement dudit dispositif de distribution de produit fluide.

Avantageusement, lesdits moyens d'ouverture comportent un élément de perçage fixe par rapport audit corps principal, adapté à découper une paroi de fermeture du réservoir de telle sorte que la ou les partie(s) découpée(s) n'obstrue(nt) pas la ou les ouverture(s) formée(s).

Avantageusement, le dispositif comporte au moins un élément de capot monté pivotant sur ledit corps principal entre une position fermée et une position ouverte.

Ces caractéristiques et avantages et d'autres de la présente invention apparaîtront plus clairement au cours de la description détaillée suivante, faite en référence aux dessins joints, donnés à titre d'exemples non limitatifs, sur lesquels:
- la figure 1 est une vue schématique en section transversale d'un dispositif de distribution selon un mode de réalisation avantageux de l'invention, en position de repos, avant ouverture et chargement du dispositif,
- la figure 2 est une vue similaire à celle de la figure 1, en position chargée, avant inhalation,
- la figure 3 est une vue similaire à celle de la figure 2, en cours d'inhalation,
- les figures 4 et 5 sont deux vues similaires à celle de la figure 3, en fin d'inhalation,
- la figure 6 est une vue similaire à celle de la figure 5, en position finale, après fermeture du dispositif,
- la figure 7 est une vue schématique en perspective partiellement découpée, en position chargée, avant inhalation,
- la figure 8 est une vue de détail de la figure 7, et
- la figure 9 est une vue schématique partielles en perspective éclatée des éléments constitutifs du dispositif de distribution des figures 1 à 8.

Sur les figures, il est représenté un exemple avantageux d'un inhalateur de poudre sèche. Sur la figure 9, seuls les éléments mentionnés dans la description ci-après comportent une référence numérique.

L'inhalateur représenté sur les figures comporte un corps principal 10 sur lequel peuvent être montées coulissantes deux parties 11, 12 formant capot, adaptées à être ouvertes pour ouvrir et charger le dispositif. Le corps principal 10 comporte deux parties de corps 10a et 10b qui s'assemblent, visibles sur la figure 9, et il peut être de forme environ arrondie comme représenté sur les figures, mais il pourrait avoir toute autre forme appropriée. Un corps supérieur 101 est assemblé au corps principal 10, et un embout buccal 200 est assemblé sur ledit corps supérieur 101. Cet embout buccal 200 définit un orifice de distribution 5 à travers lequel l'utilisateur va inhaler lors de l'actionnement du dispositif. Cet orifice de distribution 5 est typiquement disposé environ au centre de l'embout buccal 200. Les capots 11, 12 peuvent s'ouvrir par pivotement autour d'un axe de rotation commun, ou autour de deux axes 16, 17 parallèles, comme représenté sur les figures, par exemple en étant engrené l'un avec l'autre. Tout autre moyen d'ouverture du dispositif est envisageable. En variante, le dispositif pourrait ne comporter qu'un seul capot au lieu de deux.

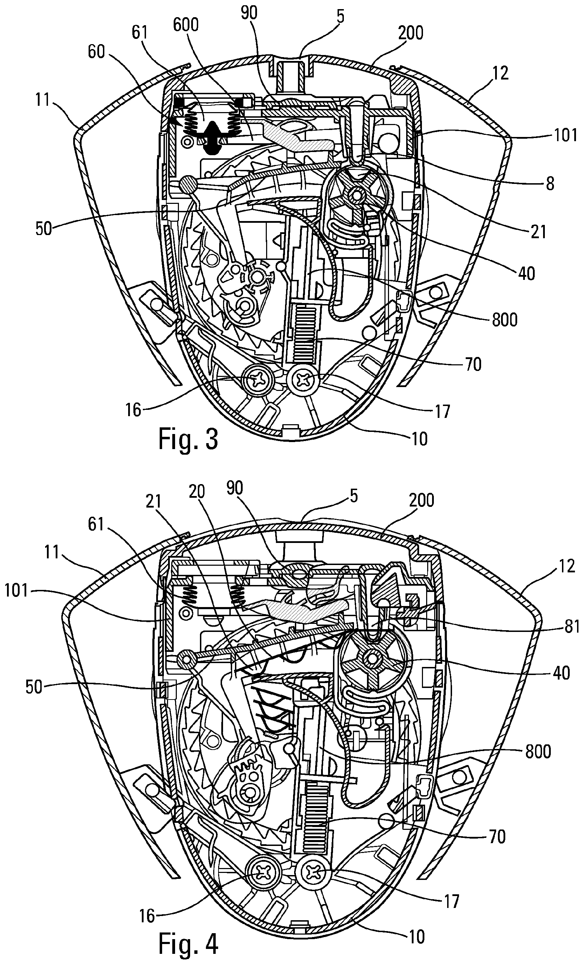

A l'intérieur du corps principal 10, il est prévu une bande 20 de réservoirs individuels 21 (visibles schématiquement seulement sur la figure 4, dans des buts de clarté), également appelés blisters, réalisée sous forme d'une bande allongée sur laquelle les blisters sont disposés les uns derrière les autres, de manière connue. Cette bande de blisters 20 est avantageusement constituée d'une couche ou paroi de base formant les cavités recevant les doses de poudre, et d'une couche ou paroi de fermeture qui obture de manière étanche chacun desdits blisters. Avant la première utilisation, la bande de blisters peut être enroulée à l'intérieur du corps principal 10, de préférence dans une partie de stockage, et des premiers moyens de déplacement de bande 40, notamment rotatifs, sont prévus pour progressivement dérouler et faire avancer cette bande de blisters.

Des seconds moyens de déplacement 50, de préférence formés par des moyens de support mobiles comportant un organe 50 pivotant sur le corps principal 10, sont prévus pour amener un blister respectif 21 dans une position de distribution à chaque actionnement du dispositif. Ces seconds moyens de déplacement sont avantageusement pivotants entre une position de non-distribution et une position de distribution, dans laquelle un blister coopère avec des moyens d'ouverture 80.

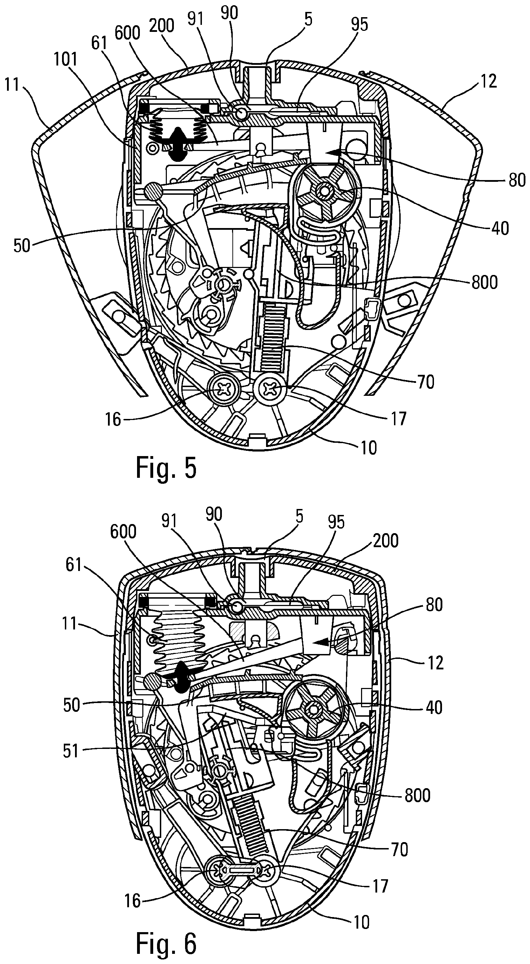

L'inhalateur comporte des moyens d'ouverture de blister 80 comportant un élément 81 de perçage et/ou de coupage de la couche de fermeture des blisters. De préférence l'élément de perçage 81 est fixe par rapport aux corps principal 10 et supérieur 101. Un blister respectif 21 est déplacé à chaque actionnement contre ledit élément de perçage 81 par les seconds moyens de déplacement 50. Le blister 21 est alors percé par ledit élément de perçage 81, qui pénètre dans ledit blister pour expulser la poudre au moyen du flux d'inhalation de l'utilisateur, comme illustré sur les figures 4 et 5. Avantageusement, les moyens d'ouverture 80 sont adaptés à découper une paroi de fermeture du réservoir ou blister 21 de telle sorte que la ou les partie(s) découpée(s) n'obstrue(nt) pas la ou les ouverture(s) formée(s). Les documents WO2006079750 et WO2009007640 décrivent de tels moyens d'ouverture de blister.

Les premiers moyens de déplacement 40 sont adaptés à faire avancer la bande de blisters après chaque inhalation de l'utilisateur. Les seconds moyens de déplacement 50 sont adaptés à déplacer le blister à vider contre lesdits moyens d'ouverture 80 lors de l'actionnement, avant chaque inhalation. Ces seconds moyens de déplacement 50 peuvent être sollicités par un élément élastique 70, tel qu'un ressort ou tout autre élément élastique équivalent, ledit élément élastique pouvant être pré-chargé lors de l'ouverture du dispositif.

De préférence, les premiers moyens de déplacement 40 sont formés par une roue d'indexage qui reçoit et guide la bande de blisters. La suite de la description sera donc faite en référence à une telle roue d'indexage 40. Une rotation de cette roue d'indexage 40 fait avancer la bande de blisters. Avant chaque inhalation, un blister plein est toujours en position face aux moyens d'ouverture 80. Les seconds moyens de déplacement 50 peuvent comporter un organe pivotant autour d'un axe de rotation, ladite roue d'indexage 40 étant avantageusement montée rotative sur ledit organe pivotant.

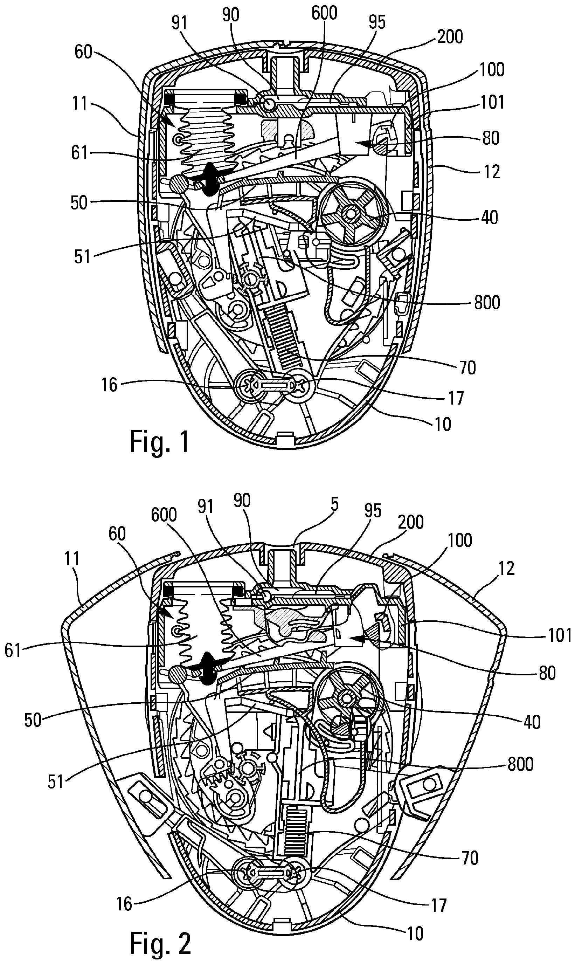

Un cycle d'actionnement du dispositif peut être le suivant. Lors de l'ouverture du dispositif, les deux parties latérales 11, 12 formant capot sont écartées l'une de l'autre en pivotant sur le corps 10 pour ouvrir le dispositif, et ainsi charger le dispositif. Dans cette position, représentée sur la figure 2, la roue d'indexage 40 ne peut pas se déplacer vers l'élément de perçage 80 car les seconds moyens de déplacement 50 sont retenus par des moyens de blocage 100 appropriés (représentés seulement très schématiquement dans des buts de clarté). Les documents WO2009077700 et WO2009136098 décrivent de tels moyens de blocage. Lors de l'inhalation par l'utilisateur à travers l'embout buccal, ces moyens de blocage 100 sont débloqués, ce qui provoque alors le déplacement de ladite roue d'indexage 40 en direction de l'élément de perçage 81, et donc l'ouverture d'un blister 21.

Comme expliqué ci-dessus, il est souhaitable que l'actionnement des moyens d'ouverture 80 soit réalisé par l'inhalation de l'utilisateur. Pour réaliser ce déclenchement par inhalation des moyens d'ouverture 80, on prévoit un système de déclenchement par l'inhalation 60 qui comporte avantageusement une chambre d'air 61 déformable sous l'effet de l'inhalation, cette chambre d'air étant adaptée à libérer les moyens de blocage 100. La chambre d'air 61 peut avantageusement être réalisée en forme de soufflet. L'inhalation de l'utilisateur provoque la déformation de ladite chambre d'air déformable 61, libérant ainsi lesdits moyens de blocage 100 et permettant donc le déplacement des seconds moyens de déplacement 50, et donc d'un blister respectif 21, vers sa position d'ouverture. Le blister 21 n'est donc ouvert qu'au moment de l'inhalation, de sorte qu'il est simultanément vidé. Il n'y a donc aucun risque de perte de dose entre l'ouverture du blister et son vidage.

L'inhalateur peut en outre comporter une chambre de dispersion 90 destinée à recevoir la dose de poudre après ouverture d'un blister respectif 21. Avantageusement, cette chambre de dispersion 90 est pourvue d'au moins une bille 91, de préférence plusieurs, qui se déplace(nt) à l'intérieur de ladite chambre de dispersion 90 pendant l'inhalation, notamment pour améliorer la distribution du mélange air et poudre après ouverture d'un blister, afin d'augmenter l'efficacité du dispositif.

Il peut être avantageux que les moyens d'ouverture 80, en particulier l'élément de perçage 81, soient directement reliés à ladite chambre de dispersion 90, par exemple via un canal 95 menant à ladite chambre 90.

Après l'inhalation, lorsque l'utilisateur referme le dispositif, tous les composants reviennent vers leurs positions de repos initiales. Le dispositif est alors prêt pour un nouveau cycle d'utilisation.

Selon un aspect avantageux de l'inhalateur, les blisters sont formés sur une bande allongée souple, qui, au début, est principalement stockée sous forme de bobine dans un logement de stockage à l'intérieur du corps principal 10 du dispositif. Avantageusement, cette bande de blisters enroulée est maintenue par des parois internes dudit logement de stockage sans que son extrémité arrière (dans le sens d'avancement de la bande de blisters) ne soit fixée par rapport audit corps principal 10, ce qui permet un assemblage plus aisé de cette bobine de bande de blisters à l'intérieur du dispositif. La bande de blisters est déplacée au moyen de la roue d'indexage 40 qui présente avantageusement au moins un, de préférence plusieurs évidements, dont la forme correspond à celle des blisters. Ainsi, lorsque cette roue d'indexage 40 tourne, elle fait avancer la bande de blisters. Bien entendu en variante ou de manière additionnelle, on pourrait utiliser d'autres moyens d'avancée de bande de blisters, par exemple en prévoyant un profil sur les bords longitudinaux latéraux de la bande de blisters, ledit profil étant adapté à coopérer avec des moyens d'entraînement appropriés. De même, des trous ménagés le long des bords latéraux de la bande de blisters pourraient également être utilisés pour faire avancer la bande de blisters au moyen de roues dentées coopérant avec lesdits trous.

Après ouverture d'un ou plusieurs blister(s), la partie de bande de blisters avec les blisters vidés doit pouvoir être stockée de manière aisée et non encombrante dans le dispositif, en évitant tout risque de blocage. Avantageusement, cette bande de blisters usée s'enroule automatiquement sur elle-même pour à nouveau former une bobine.

L'élément de capot mobile 12 est solidaire d'un organe d'armement 800 qui peut coulisser dans un logement approprié. Cet organe d'armement 800 est avantageusement pivotant par rapport audit corps 10 ensemble avec l'élément de capot 12. Cet organe d'armement 800 peut se déplacer contre le ressort 70, avantageusement un ressort à boudins, disposé dans ledit logement. L'organe d'armement 800 est donc connecté d'un côté audit ressort 70 et de l'autre côté, il coopère avec les seconds moyens de déplacement, en particulier avec un organe 50 pivotant sur le corps 10, et sur lequel est fixée de manière rotative la roue d'indexage 40.

Lorsque l'élément de capot mobile 12 est ouvert, l'organe d'armement 800 est déplacé dans son logement en comprimant le ressort 70. L'organe pivotant 50 des seconds moyens de déplacement est lui empêché de se déplacer par les moyens de blocage 100 susmentionnés, qui ne seront libérés qu'au moment de l'inhalation. Ainsi, en l'absence d'inhalation dans la position ouverte, la fermeture des éléments de capot 11, 12 provoquerait simplement le retour à la position de repos pour l'organe d'armement 800, et la décompression du ressort 70.

Ainsi, lorsque l'utilisateur ouvre l'inhalateur il charge le système. S'il n'inhale pas et qu'il referme l'inhalateur, celui-ci reprend simplement sa position de départ sans déplacer la bande de blisters ni les moyens de blocage. Il n'y a donc aucun risque qu'un blister (et donc une dose de produit actif) soit perdu par un actionnement malencontreux ou incomplet dans lequel l'utilisateur n'exercerait pas d'inhalation entre l'ouverture et la fermeture. L'ouverture du blister, son vidage, la distribution de la poudre dans les poumons de l'utilisateur, le déplacement de la bande de blisters pour amener un nouveau blister plein en face des moyens d'ouverture ainsi que le comptage de la dose ne sont donc possibles que si l'utilisateur inhale.

Les moyens de blocage 100, qui bloquent les seconds moyens de déplacement et notamment l'organe pivotant 50 qui coopère avec l'organe d'armement 800, sont connectés à la chambre d'air déformable 61 sensible à l'inhalation de l'utilisateur via un élément déclencheur 600, de sorte que lors de l'inhalation de l'utilisateur, ladite chambre d'air déformable 61 se déforme, faisant pivoter ledit élément déclencheur 600 et libérant lesdits moyens de blocage 100. Ceci permet le déplacement desdits seconds moyens de déplacement 50 vers leur position de distribution sous l'effet de la force exercée par le ressort comprimé 70 sur l'organe d'armement 800 qui pousse sur l'organe pivotant 50. Ce déplacement provoque l'ouverture d'un blister plein et la distribution d'une dose.

Une surface de came 51 est formée sur lesdits moyens de support mobiles 50, sur laquelle glisse l'organe d'armement 800. L'organe d'armement 800 est donc adaptée à comprimer le ressort 70 lorsque l'élément de capot 12 est ouvert, et à décomprimer ledit ressort 70 lorsque ledit élément de capot 12 est refermé.

Avantageusement, l'organe d'armement 800 comporte, dans sa partie en contact avec la surface de came 51, une partie arrondie 801 pour favoriser le glissement de l'organe d'armement 800 sur ladite surface de came 51.

Les moyens de support mobiles sont dans ce mode de réalisation réalisés sous la forme d'un organe 50 monté pivotant sur le corps 10 autour d'un axe de pivotement. La surface de came précitée 51 étant formée sur ledit organe pivotant 50, lorsque le ressort 70 est chargé lors de l'ouverture de l'élément de capot mobile 12, ledit organe pivotant 50 est sollicité vers sa position de distribution par ledit organe d'armement 800 et le ressort 70 comprimé.

La surface de came 51 peut comporter au moins deux parties de pente différentes, avantageusement séparées par un sommet. En partant à nouveau de la position fermée de l'élément de capot mobile, la première partie de pente sur laquelle coulisse l'organe d'armement 800 permet la compression du ressort 70 comme décrit précédemment. Lorsque le ressort est chargé, c'est-à-dire comprimé, la surface de came 51 peut prévoir une seconde partie de pente différente avec laquelle l'organe d'armement 800 va coopérer uniquement en position d'ouverture du dispositif. De préférence, l'organe d'armement 800 exerce une force sensiblement perpendiculaire sur cette seconde partie de surface de came. De cette manière, la position chargée est stable. En variante, la seconde partie de pente forme un cran de butée dans laquelle l'organe d'armement 800 vient se positionner en position ouverte.

Après l'inhalation, c'est-à-dire en position de distribution, les moyens de blocage ont été libérés et les moyens de support mobiles 50 ont été déplacés vers le haut par le ressort comprimé 70.

Avantageusement, les deux éléments de capot mobiles 11, 12 sont engrenés l'un dans l'autre via des engrenages appropriés pour garantir une ouverture et une fermeture symétrique desdits deux éléments de capot mobiles. Cet engrenage peut se faire à proximité de leurs axes de pivotement 16, 17.

L'embout buccal 200 est de préférence symétrique par rapport audit orifice de distribution 5, comme visible sur la figure 9. Il comporte une paroi supérieure 210 qui s'étend longitudinalement des deux côtés de l'orifice de distribution 5. On constate que cette paroi supérieure est légèrement bombée ou convexe dans la direction longitudinale, octroyant ainsi à l'embout buccal 200 une forme arrondie aplatie. De part et d'autre de l'orifice de distribution 5, dans la direction transversale ou latérale, l'embout buccal comporte deux surfaces d'appui latérales 220 respectivement prévues latéralement de chaque côté dudit orifice de distribution 5. Chacune de ces surfaces d'appui latérales 220 est destinée à recevoir une lèvre de l'utilisateur lors de l'inhalation. Ces surfaces d'appui latérales 220 sont légèrement concaves, notamment dans la direction transversale, pour former de légers creux adaptés à bien positionner les lèvres de l'utilisateur lorsque celui-ci les place sur l'embout buccal. Comme visible sur la figure 9, les surfaces d'appui latérales 220 prolongent latéralement ladite paroi supérieure 210, et sont de préférence séparées l'une de l'autre par cette paroi supérieure 210, ainsi que par l'orifice de distribution 5. De cette manière, lorsque l'utilisateur place sa bouche sur l'embout buccal 200, l'écartement entre les deux surfaces d'appui latérales 220 oblige l'utilisateur à ouvrir suffisamment la bouche pour éviter que ses dents ne viennent obturer même partiellement l'orifice de distribution 5 lors de l'inhalation. Par ailleurs, la forme légèrement concave des surfaces d'appui latérales 220 garantit une bonne ergonomie et assure une bonne étanchéité de la bouche de l'utilisateur sur l'embout buccal 200 au moment de l'inhalation. Ceci évite notamment une perte de charge lors de l'inhalation et garantit que la plus grande partie possible de l'écoulement d'inhalation crée par l'utilisateur sera utilisée pour actionner l'inhalateur et expulser la poudre contenue dans l'inhalateur. L'écartement et la profondeur des surfaces d'appui latérales 220 garantit également que l'orifice de distribution se positionne à l'intérieur de la bouche, au-delà des dents, sans pour autant que cet orifice de distribution 5 soit formé sur une partie saillante de l'embout buccal 200.

Avantageusement, les surfaces d'appui latérales 220 s'étendent longitudinalement sur une partie centrale dudit embout buccal 200, de préférence sur la plus grande partie de celui-ci, comme cela est notamment visible sur la figure 9. Avantageusement, ces deux surfaces d'appui latérales 220 sont parfaitement symétriques l'une par rapport à l'autre, par rapport à l'orifice de distribution 5.

Avantageusement, les éléments de capot 11, 12 mobiles autour du corps principal 10 de l'inhalateur et de l'embout buccal 200, comportent une surface supérieure de forme bombée sensiblement adaptée à celle de l'embout buccal, notamment à sa paroi supérieure 210. De cette manière, lors du déplacement desdits éléments de capot entre la position d'ouverture et de fermeture, aucun espace n'est crée par la présence de l'embout buccal 200, de sorte que l'utilisateur ne risque pas de se coincer les doigts lors de la manipulation de ces éléments de capot. De manière générale, l'embout buccal ne comporte aucune partie saillante qui risquerait de créer un interstice pouvant recevoir des doigts de l'utilisateur lors de l'actionnement du dispositif.

Selon l'invention, il est prévu un dispositif électronique de comptage ou d'indication de doses 300. Ce dispositif peut comporter un écran affichant des chiffres ou symboles visibles à travers une fenêtre appropriée dans le corps principal 10 du dispositif.

Un but de l'invention est d'éviter de compter des doses qui ne sont pas distribuées, par exemple en cas d'erreur de manipulation, ou d'une manipulation incomplète du dispositif. Il est donc souhaitable que le compteur ou indicateur ne soit actionné qu'en cas d'inhalation, puisque c'est cette inhalation qui conditionne l'ouverture et la distribution de la dose de chaque blister. Avantageusement, l'actionnement du compteur se fait donc lors ou après l'inhalation.

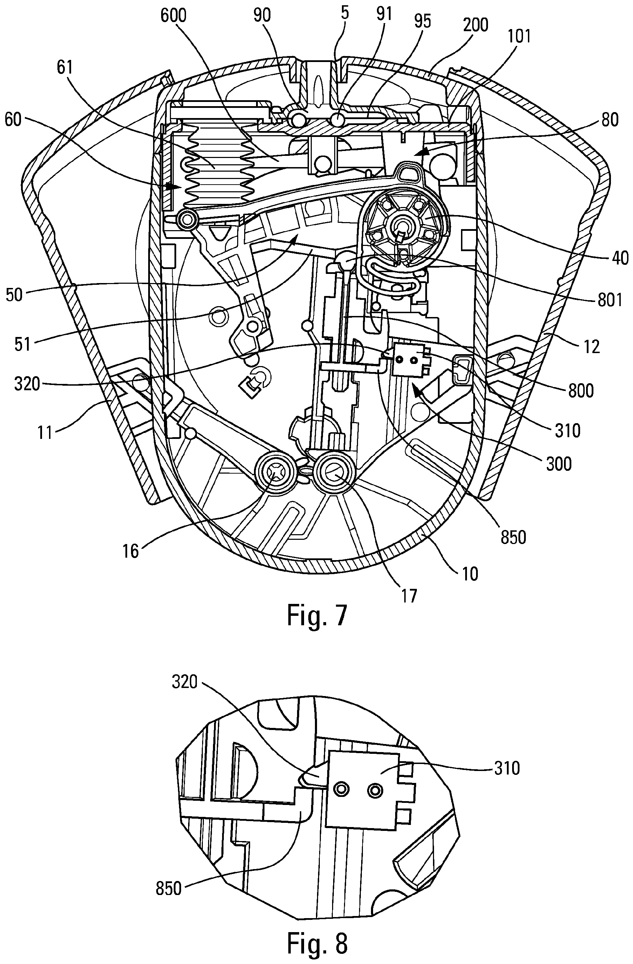

Les figures 7 à 9 illustrent un mode de réalisation avantageux du dispositif électronique de comptage ou d'indication de doses 300. Dans cet exemple, le dispositif électronique de comptage ou d'indication de doses 300 comporte un corps 301 et un couvercle 302 pourvu d'une fenêtre 303 obturée par une paroi transparente 304. Cette mise en œuvre n'est toutefois pas limitative, et le compteur 300 pourrait simplement être logé dans une partie appropriée du corps 10 de l'inhalateur.

Le dispositif électronique de comptage ou d'indication de doses 300 comporte un écran 305, notamment du type LCD (cristaux liquides), un capteur 306, une source d'alimentation 307, telle qu'une pile ou un accumulateur, et un circuit imprimé 308. Il est à noter que ces différents éléments pourraient être réalisés différemment de ce qui est représenté à titre d'exemple sur les dessins.

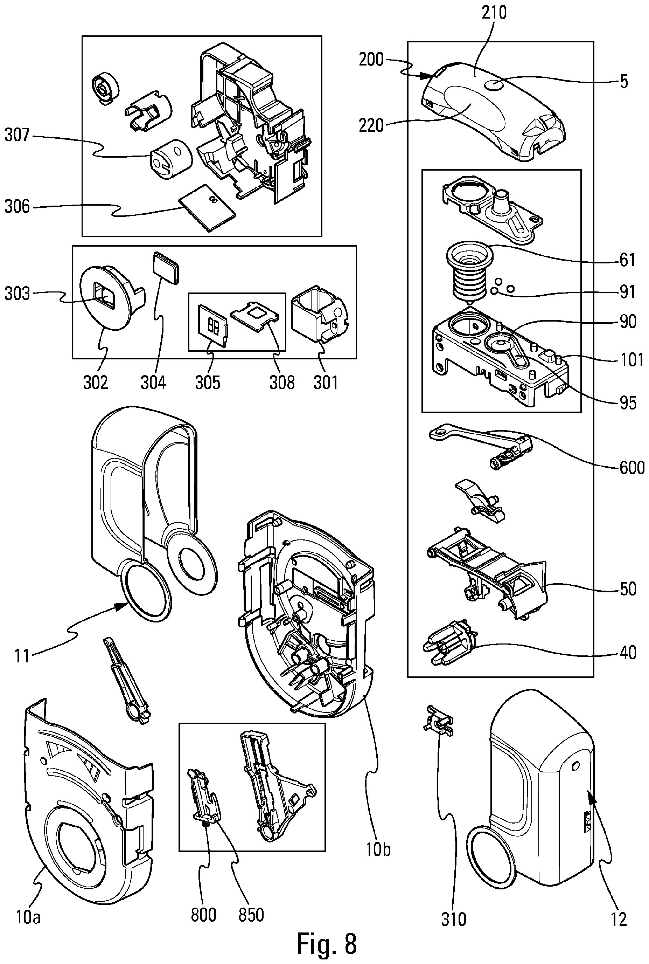

Avantageusement, on prévoit un actionneur 310, pourvu d'un connecteur 320. L'organe d'armement 800 comporte alors avantageusement une extension 850 adaptée à coopérer dans la position chargée de l'organe d'armement 800 avec ledit connecteur pour activer le dispositif électronique de comptage ou d'indication de doses 300. Ainsi, avant ouverture de l'inhalateur et chargement de l'organe d'armement 800, le dispositif électronique de comptage ou d'indication de doses 300 est de préférence en veille pour économiser la source d'alimentation 307. Lorsque l'inhalateur est ouvert et l'organe d'armement 800 chargé, l'extension 850 de l'organe d'armement 800 coopère avec le connecteur 320 de l'actionneur 310, ce qui va activer le dispositif électronique de comptage ou d'indication de doses 300, comme visible sur les figures 7 et 8.

A ce stade, si l'utilisateur referme l'inhalateur sans inhaler, le dispositif électronique de comptage ou d'indication de doses 300 ne sera pas actionné mais simplement désactivé. Aucune dose ne sera comptée.

Si par contre l'utilisateur inhale, le dispositif électronique de comptage ou d'indication de doses 300 est actionné pour compter une dose et l'afficher sur l'écran 305.

Selon l'invention, l'actionnement du compteur est donc corrélé au déplacement des moyens de support mobiles (50) de l'inhalateur, réalisé lors ou après l'inhalation. En effet, dès lors que l'utilisateur inhale à travers l'embout buccal, la distribution d'une dose de poudre est inévitable, et doit donc être comptée par le compteur.

Ainsi, le capteur 306 peut détecter le mouvement des moyens de support mobiles 50 qui déplacent le réservoir à vider contre l'élément de perçage 81 après l'inhalation.

En variante, le capteur 306 pourrait détecter le retour de ces moyens de support mobiles 50, après vidage du réservoir par l'inhalation.

Dans une autre variante (non revendiquée, et qui ne fait pas partie de l'objet de la présente invention), le capteur 306 pourrait détecter la libération des moyens de blocage 100 et/ou le déplacement/déformation de l'élément déclencheur 600.

Encore une autre variante (non revendiquée, et qui ne fait pas partie de l'objet de la présente invention) serait de détecter le déplacement/déformation de la chambre d'air déformable 61.

Enfin, lorsque les premiers moyens de déplacement sont actionnés après inhalation, notamment lors de la fermeture de l'inhalateur, alors le capteur 306 pourrait aussi détecter ce déplacement, en particulier la rotation de la roue de guidage 40.

Le capteur 306, lorsqu'il est ainsi actionné, transmet un signal au circuit imprimé, qui va commander la modification de l'affichage de l'écran 305.

Ce signal peut aussi être stocké dans une mémoire appropriée, ensemble avec des informations d'horodatage et/ou de géolocalisation. Avantageusement, le dispositif comporte des moyens de transmission de données sans fil, notamment du type Bluetooth®, en particulier Bluetooth® à basse énergie (BLE), pour transmettre ces informations, par exemple à un smartphone ou similaire.

Il est à noter que d'autres capteurs pourraient être associés au capteur 306, par exemple du type accéléromètre, pour détecter l'orientation et/ou les mouvements de l'utilisateur lors de l'utilisation de l'inhalateur, afin de compléter les informations relatives au comptage de dose.

La présente invention permet donc de fournir un inhalateur de poudre sèche qui procure notamment les fonctions suivantes :
▪ une pluralité de doses individuelles de poudre stockées dans des blisters individuels étanches, par exemple 30 ou 60 doses stockées sur une bande enroulée en bobine ;
▪ la poudre libérée au moyen d'un perçage actionné par l'inhalation de l'utilisateur, ce perçage du blister étant réalisé au moyen d'un système de détection d'inhalation couplé à un système de libération pré-chargé ;
▪ des moyens d'entraînement de forme appropriée en prises avec les blisters pour réaliser le déplacement de la bande de blisters après chaque inhalation, et amener un nouveau blister plein dans une position dans laquelle il est destiné à être ouvert par les moyens d'ouverture appropriés ;
▪ des moyens pour éviter une perte de dose en cas d'ouverture de l'inhalateur mais en l'absence d'inhalation ;
▪ a un indicateur de doses fiable adapté à compter les doses seulement en cas d'inhalation.

D'autres fonctions sont également fournies par le dispositif de l'invention tel que cela a été décrit précédemment.

Diverses modifications sont également possibles pour un homme du métier sans sortir du cadre de la présente invention tel que défini par les revendications annexées.

## Revendications

1. Dispositif de distribution de produit fluide, comportant un corps principal (10), ledit dispositif comportant:
- au moins un réservoir individuel (21) contenant une dose unique de produit fluide, tel que de la poudre,
- des moyens d'ouverture (80) pour ouvrir un réservoir individuel à chaque actionnement du dispositif,
- un embout buccal (200) définissant un orifice de distribution (5),
- des moyens de support mobiles (50) adaptés à déplacer un réservoir individuel (21) contre lesdits moyens d'ouverture (80) à chaque actionnement, lesdits moyens de support mobiles (50) étant déplaçables entre une position de non-distribution et une position de distribution, lesdits moyens de support mobiles (50) étant sollicités vers leur position de distribution par des moyens élastiques (70), tel qu'un ressort ou une lame élastique, et étant retenus en position de non-distribution par des moyens de blocage (100),
- un système de déclenchement par l'inhalation (60) qui comporte une chambre d'air déformable (61) coopérant avec ledit orifice de distribution (5), un élément déclencheur (600) coopérant d'une part avec ladite chambre d'air (61) et d'autre part avec lesdits moyens de blocage (100), de sorte que lors d'une inhalation à travers ledit orifice de distribution (5), ladite chambre d'air (61) est déformée et ledit élément déclencheur (600) libère lesdits moyens de blocage (100), de sorte que lors d'une inhalation, un réservoir est déplacé contre et ouvert par lesdits moyens d'ouverture (80),
**caractérisé en ce que** ledit dispositif de distribution de produit fluide comporte un dispositif électronique de comptage ou d'indication de doses (300) comprenant un capteur (306) adapté à détecter le déplacement lors ou après l'inhalation de l'utilisateur desdits moyens de support mobiles (50).

2. Dispositif selon la revendication 1, dans lequel ledit capteur (306) est actionné par lesdits moyens de support mobiles (50) lors de leur déplacement entre ladite position de non-distribution et ladite position de distribution.

3. Dispositif selon la revendication 1, dans lequel ledit capteur (306) est actionné par lesdits moyens de support mobiles (50) lors de leur déplacement entre ladite position de distribution et ladite position de non-distribution.

4. Dispositif selon l'une quelconque des revendications précédentes, comportant une bande allongée (20) de réservoirs individuels (21) coopérant avec des premiers moyens de déplacement (40) adaptés à faire avancer ladite bande après chaque actionnement, lesdits premiers moyens de déplacement comportant une roue de guidage (40) montée rotative sur lesdits moyens de support mobiles (50).

5. Dispositif selon la revendication 4, dans lequel ledit capteur (306) est actionné par la rotation de ladite roue de guidage (40).

6. Dispositif selon l'une quelconque des revendications précédentes, dans lequel ledit dispositif électronique de comptage ou d'indication de doses (300) comprend un écran (305), notamment du type LCD, une source d'alimentation (307), telle qu'une pile ou un accumulateur, et un circuit imprimé (308).

7. Dispositif selon la revendication 6, dans lequel ledit capteur (306), lorsqu'il est actionné, transmet un signal audit circuit imprimé (308), qui va commander la modification de l'affichage dudit écran (305).

8. Dispositif selon l'une quelconque des revendications précédentes, comportant un organe d'armement (800) déplacé vers une position chargée lors de l'ouverture du dispositif de distribution de produit fluide, ledit dispositif électronique de comptage ou d'indication de doses (300) comprenant un actionneur (310) pourvu d'un connecteur (320), et ledit organe d'armement (800) comportant une extension (850) adaptée à coopérer, dans la position chargée dudit organe d'armement (800), avec ledit connecteur (320) pour activer le dispositif électronique de comptage ou d'indication de doses (300).

9. Dispositif selon l'une quelconque des revendications précédentes, dans lequel ledit dispositif électronique de comptage ou d'indication de doses (300) comprend une mémoire adaptée à stocker les informations générées par ledit capteur (306).

10. Dispositif selon l'une quelconque des revendications précédentes, dans lequel ledit dispositif électronique de comptage ou d'indication de doses (300) comprend des moyens de transmission de données sans fil, notamment du type Bluetooth®, en particulier Bluetooth® à basse énergie (BLE).

11. Dispositif selon l'une quelconque des revendications précédentes, dans lequel ledit dispositif électronique de comptage ou d'indication de doses (300) comprend d'autres capteurs associés audit capteur (306), notamment du type accéléromètre, pour détecter l'orientation et/ou les mouvements de l'utilisateur lors de l'actionnement dudit dispositif de distribution de produit fluide.

12. Dispositif selon l'une quelconque des revendications précédentes, dans lequel lesdits moyens d'ouverture (80) comportent un élément de perçage (81) fixe par rapport audit corps principal (10), adapté à découper une paroi de fermeture du réservoir (21) de telle sorte que la ou les partie(s) découpée(s) n'obstrue(nt) pas la ou les ouverture(s) formée(s).

13. Dispositif selon l'une quelconque des revendications précédentes, comportant au moins un élément de capot (11, 12) monté pivotant sur ledit corps principal (10) entre une position fermée et une position ouverte.

## Patentansprüche

1. Vorrichtung zum Verteilen eines Fluidprodukts, die einen Hauptkörper (10) besitzt und Folgendes umfasst:
- wenigstens einen individuellen Vorratsbehälter (21), der eine einzige Dosis des Fluidprodukts wie etwa ein Pulver enthält,
- Öffnungsmittel (80) zum Öffnen eines individuellen Vorratsbehälters bei jeder Betätigung der Vorrichtung,
- ein Mundstück (200), das eine Verteilungsöffnung (5) definiert,
- bewegliche Tragmittel (50), die dafür ausgelegt sind, einen individuellen Vorratsbehälter (21) bei jeder Betätigung zu den Öffnungsmitteln (80) zu verlagern, wobei die beweglichen Tragmittel (50) zwischen einer Nichtverteilungsposition und einer Verteilungsposition verlagerbar sind, wobei die beweglichen Tragmittel (50) durch elastische Mittel (70) wie etwa eine Feder oder ein elastisches Plättchen in ihre Verteilungsposition vorbelastet sind und in der Nichtverteilungsposition durch Blockiermittel (100) gehalten werden,
- ein System (60) zum Auslösen durch Einatmen, das eine verformbare Luftkammer (61), die mit der Verteilungsöffnung (5) zusammenwirkt, und ein Auslöseelement (600), das einerseits mit der Luftkammer (61) und andererseits mit den Blockiermitteln (100) zusammenwirkt, umfasst, derart, dass bei einem Einatmen durch die Verteilungsöffnung (5) die Luftkammer (61) verformt wird und das Auslöseelement (600) die Blockiermittel (100) freigibt, so dass bei einem Einatmen der Vorratsbehälter zu den Öffnungsmitteln (80) verlagert und durch diese geöffnet wird,
**dadurch gekennzeichnet, dass** die Vorrichtung zum Verteilen eines Fluidprodukts eine elektronische Vorrichtung (300) zum Zählen oder Angeben von Dosen umfasst, die einen Zähler (306) enthält, der dafür ausgelegt ist, die Verlagerung während oder nach dem Einatmen des Benutzers der beweglichen Tragmittel (50) zu detektieren.

2. Vorrichtung nach Anspruch 1, wobei der Sensor (306) durch die beweglichen Tragmittel (50) betätigt wird, wenn diese zwischen der Nichtverteilungsposition und der Verteilungsposition verlagert werden.

3. Vorrichtung nach Anspruch 1, wobei der Sensor (306) durch die beweglichen Tragmittel (50) betätigt wird, wenn diese zwischen der Verteilungsposition und der Nichtverteilungsposition verlagert werden.

4. Vorrichtung nach einem der vorhergehenden Ansprüche, die einen länglichen Streifen (20) individueller Vorratsbehälter (21) umfasst, der mit ersten Verlagerungsmitteln (40) zusammenwirkt, die dafür ausgelegt sind, das Band bei jeder Betätigung vorwärts zu bewegen, wobei die ersten Verlagerungsmittel ein Führungsrad (40) aufweisen, das an den beweglichen Tragmitteln (50) drehbar montiert ist.

5. Vorrichtung nach Anspruch 4, wobei der Sensor (306) durch die Drehung des Führungsrades (40) betätigt wird.

6. Vorrichtung nach einem der vorhergehenden Ansprüche, wobei die elektronische Vorrichtung (300) zum Zählen oder Angeben von Dosen einen Bildschirm (305) insbesondere des LCD-Typs, eine Versorgungsquelle (307) wie etwa eine Batterie oder einen Akkumulator und eine gedruckte Schaltung (308) umfasst.

7. Vorrichtung nach Anspruch 6, wobei der Sensor (306) dann, wenn er betätigt wird, an die gedruckte Schaltung (308) ein Signal überträgt, damit diese die Veränderung der Anzeige des Bildschirms (305) steuert.

8. Vorrichtung nach einem der vorhergehenden Ansprüche, die ein Spannorgan (800) umfasst, das beim Öffnen der Vorrichtung zum Verteilen des Fluidprodukts in eine gespannte Position verlagert wird, wobei die elektronische Vorrichtung (300) zum Zählen oder Angeben von Dosen einen Aktor (310) enthält, der mit einem Verbinder (320) versehen ist, und wobei das Spannorgan (800) eine Verlängerung (850) besitzt, die dafür ausgelegt ist, in der gespannten Position des Spannorgans (800) mit dem Verbinder (320) zusammenzuwirken, um die elektronische Vorrichtung (300) zum Zählen oder Angeben von Dosen zu aktivieren.

9. Vorrichtung nach einem der vorhergehenden Ansprüche, wobei die elektronische Vorrichtung (300) zum Zählen oder Angeben von Dosen einen Speicher enthält, der dafür ausgelegt ist, die durch den Sensor (306) erzeugten Informationen zu speichern.

10. Vorrichtung nach einem der vorhergehenden Ansprüche, wobei die Vorrichtung zum Zählen oder Angeben von Dosen (300) Mittel zum drahtlosen Übertragen von Daten insbesondere des Bluetooth®-Typs, speziell des Niedrigenergie-Bluetooth®-Typs (BLE-Typ), umfasst.

11. Vorrichtung nach einem der vorhergehenden Ansprüche, wobei die Vorrichtung (300) zum Zählen oder Angeben von Dosen weitere Sensoren, insbesondere des Beschleunigungsmesse-Typs, umfasst, die dem Sensor (306) zugeordnet sind, um die Orientierung und/oder die Bewegungen des Benutzers bei der Betätigung der Vorrichtung zum Verteilen eines Fluidprodukts zu detektieren.

12. Vorrichtung nach einem der vorhergehenden Ansprüche, wobei die Öffnungsmittel (80) ein Durchlochungselement (81) aufweisen, das in Bezug auf den Hauptkörper (10) fest ist und dafür ausgelegt ist, eine Wand zum Verschließen des Vorratsbehälters (21) in der Weise zu zerschneiden, dass der eine oder die mehreren zerschnittenen Teile die eine oder die mehreren gebildeten Öffnungen nicht blockieren.

13. Vorrichtung nach einem der vorhergehenden Ansprüche, die wenigstens ein Abdeckelement (11, 12) umfasst, das an dem Hauptkörper (10) drehbar zwischen einer geschlossenen Position und einer geöffneten Position montiert ist.

## Claims

1. A fluid dispenser device including a main body (10), said device comprising:
▪ at least one individual reservoir (21) containing a single dose of fluid, such as powder,
▪ opening means (80) for opening an individual reservoir each time the device is actuated,
▪ a mouthpiece (200) defining a dispenser orifice (5),
▪ movable support means (50) that are adapted to displace an individual reservoir (21) against said opening means (80) on each actuation, said movable support means (50) being displaceable between a non-dispensing position and a dispensing position, said movable support means (50) being urged towards their dispensing position by resilient means (70), such as a spring or a spring blade, and being held in their non-dispensing position by blocking means (100),
▪ an inhalation trigger system (60) that comprises a deformable air chamber (61) that co-operates with said dispenser orifice (5), and a trigger element (600) that co-operates firstly with said air chamber (61) and secondly with said blocking means (100), so that during inhalation through said dispenser orifice (5), said air chamber (61) is deformed and said trigger element (600) releases said blocking means (100), so that during inhalation, a reservoir is displaced against said opening means (80) and is opened by said opening means (80),
said fluid dispenser device being **characterized in that** it further comprises an electronic dose counter or indicator device (300) including a sensor (306) that is adapted to detect the displacement of said movable support means (50) while the user is inhaling or after the user has inhaled.

2. A device according to claim 1, wherein said sensor (306) is actuated by said movable support means (50) while they are being displaced between said non-dispensing position and said dispensing position.

3. A device according to claim 1, wherein said sensor (306) is actuated by said movable support means (50) while they are being displaced between said dispensing position and said non-dispensing position.

4. A device according to any preceding claim, including an elongate strip (20) of individual reservoirs (21) co-operating with first displacement means (40) that are adapted to cause said strip to advance after each actuation, said first displacement means comprising a guide wheel (40) that is rotatably mounted on said movable support means (50).

5. A device according to claim 4, wherein said sensor (306) is actuated by said guide wheel (40) turning.

6. A device according to any preceding claim, wherein said electronic dose counter or indicator device (300) comprises a screen (305), in particular of the LCD type, a power supply (307), such as an optionally rechargeable battery, and a printed circuit (308).

7. A device according to claim 6, wherein said sensor (306), when it is actuated, transmits a signal to said printed circuit (308), which signal causes the display on said screen (305) to be changed.

8. A device according to any preceding claim, including a cocking member (800) that is displaced towards a cocked position while opening the fluid dispenser device, said electronic dose counter or indicator device (300) including an actuator (310) that is provided with a connector (320), and said cocking member (800) including an extension (850) that is adapted to cooperate, in the cocked position of said cocking member (800), with said connector (320) so as to activate the electronic dose counter or indicator device (300).

9. A device according to any preceding claim, wherein said electronic dose counter or indicator device (300) includes a memory that is adapted to store the information generated by said sensor (306).

10. A device according to any preceding claim, wherein said electronic dose counter or indicator device (300) includes wireless data transmission means, in particular of the Bluetooth® type, in particular of the Bluetooth® low energy (BLE) type.

11. A device according to any preceding claim, wherein said electronic dose counter or indicator device (300) includes other sensors associated with said sensor (306), in particular sensors of the accelerometer type, for detecting the
orientation and/or the movements of the user while said fluid dispenser device is being actuated.

12. A device according to any preceding claim, wherein said opening means (80) include a perforator element (81) that is stationary relative to said main body (10), adapted to cut a closure wall of the reservoir (21) in such a manner that the cut portion(s) does/do not obstruct the opening(s) that is/are formed.

13. A device according to any preceding claim, including at least one cover element (11, 12) that is mounted to pivot on said main body (10) between a closed position and an open position.
